# EUROPÄISCHE PATENTSCHRIFT

(11) **EP 0 505 353 B1**
(45) Veröffentlichungstag und Bekanntmachung des Hinweises auf die Patenterteilung: **08.09.1993**
(21) Anmeldenummer: 89913036.3
(22) Anmeldetag: 01.12.1989
(51) Int. Cl.: G03G 15/00, G03G 15/16

(54) **SCHNELLDRUCKEINRICHTUNG MIT EINER IM PAPIERKANAL ANGEORDNETEN PARTIKELFALLE**
FAST PRINTER WITH A PARTICLE TRAP IN THE PAPER CHANNEL
IMPRIMANTE RAPIDE AVEC PIEGE A PARTICULES AGENCE DANS LE PASSAGE A PAPIER

(30) Priorität: 05.04.1989 DE 3911026
(43) Veröffentlichungstag der Anmeldung: 30.09.1992
(73) Patentinhaber: Siemens Nixdorf Informationssysteme Aktiengesellschaft, 33102 Paderborn (DE)
(72) Erfinder: RUMPEL, Peter, D-8152 Feldkirchen (DE)
(74) Vertreter: Fuchs, Franz-Josef, Dr.-Ing.
(86) Internationale Anmeldenummer: DE8900747
(87) Internationale Veröffentlichungsnummer: WO9012344

(56) Entgegenhaltungen:
- PATENT ABSTRACTS OF JAPAN, vol. 10, No. 93 (P-445)(2150), 10 April 1986, & JP-A-60229038
- PATENT ABSTRACTS OF JAPAN, vol. 10, No. 341 (P-517)(2397), 18 November 1986, & JP-A-61144666
- XEROX DISCLOSURE JOURNAL, vol. 1, No. 6, June 1976, (Stamford, Connecticut, US), C.M. Cavitt : "Magnetic guard against debris in paper tray", page 63
- PATENT ABSTRACTS OF JAPAN, vol. 8, No. 192 (P-298)(1629), 4 September 1984, & JP-A-5979274
- XEROX DISCLOSURE JOURNAL, vol. 10, No. 6, November/December 1985, (Stamford, Connecticut, US), I.F. Palumbo : "Magnetic Devices for Capturing Foreign Metallic Objects", page 347
- PATENT ABSTRACTS OF JAPAN, Vol. 9, No. 210 (P-383)(1933), 28 August 1985, & JP-A-6070458

## Beschreibung

Die Erfindung betrifft eine Schnelldruckeinrichtung gemäß dem Oberbegriff des Patentanspruches 1.

Als moderne Schnelldruckeinrichtungen werden im allgemeinen nichtmechanische Druckgeräte verwendet, wie z.B. Laserdrucker, mit LED-Kämmen arbeitende Drucker, magnetische Drucker oder mit Tintendruck arbeitende Drucker. Diese sind allgemein bekannt und mit Erfolg zur Anwendung gelangt.

Bei nach dem Prinzip der Elektrofotografie arbeitenden Druckern wird mit Hilfe einer zeichenabhängig gesteuerten Lichtquelle auf einer Fotoleitertrommel ein latentes Bild erzeugt, das durch Auftragen von Toner in einer Entwicklerstation eingefärbt und dann in einer nachfolgenden Umdruckstation auf einen Aufzeichnungsträger übertragen wird. Das sich lose auf dem Aufzeichnungsträger befindliche, aus Toner bestehende Bild wird dann mit Hilfe einer Fixiereinrichtung fixiert.

Die als Zwischenträger verwendeten Fotoleitertrommeln von elektrofotografischen Druckern, seien sie nun aus anorganischem oder organischem Material, weisen eine empfindliche Oberfläche auf, die leicht zerkratzt werden kann. Evtl. mit dem Aufzeichnungsträger mitgeführte Metallpartikeln, z.B. Büroklammern, können die Oberfläche der Fotoleitertrommel beschädigen, was zu einer nicht mehr korrigierbaren Störung des Druckbildes führten.

Auch bei nichtmechanischen Druckeinrichtungen die mit Tintendruck arbeiten, sei es nun unter Verwendung eines zeilenweise bewegten Kopfes oder eines Tintenkammes, kann infolge des erforderlichen dichten Abstandes zwischen Tintenaustrittsdüsen und Aufzeichnungsträger eine mit dem Aufzeichnungsträger mitgeführte Partikel Störungen des Schreibbetriebes hervorrufen oder den Tintenkopf oder Tintenkamm beschädigen.

Um das Eindringen von Partikeln zu verhindern ist es deswegen üblich bei Schnelldruckeinrichtungen jeder Art die Papierzuführung abzudecken bzw. zu kapseln.

Schnelldruckeinrichtungen werden im allgemeinen jedoch zusammen mit EDV-Anlagen betrieben und vom gleichen Bedienungspersonal bedient. Aus diesem Grunde müssen die Druckeinrichtungen so ausgestaltet sein, daß ein nahezu unterbrechungsfreier Druckbetrieb gewährleistet ist. So muß nach Verbrauch eines Papierstapels ohne wesentliche Unterbrechungszeit sehr schnell ein neuer Stapel eingelegt werden können. Aus diesem Grunde ist es notwendig, die Druckeinrichtung ergonomisch optimal auszugestalten, dies gilt insbesondere für das Einlegen und Herausnehmen des Papieres und für die Wartung der Gesamtanlage. Aus diesem. Grunde muß zum Einlegen des Aufzeichnungsträgers die Druckeinrichtung weit geöffnet werden können. Damit besteht die Gefahr, daß beim Einlegen Partikeln wie Büroklammern oder dergleichen in den Bereich des Aufzeichnungsträgers gelangen können und dort z.B. durch elektrostatische Kräfte anhaften.

Um diese Partikeln abfangen zu können, ist es aus den Literaturstellen JP-A-60-229 038 (Abstract) und Xerox Discl. Journal, Band 1, Nr. 6, Seite 63 bekannt, Partikelfallen anzuordnen, die Partikeln magnetisch abfangen.

Es ist weiterhin aus der Literaturstelle JP-A-61-144 666 bekannt, in den Papierkanal eingedrungene Partikel mit Hilfe einer Elektrode elektrisch zu detektieren und gegebenenfalls den Papiertransport abzuschalten.

Aufgabe der Erfindung ist es für Druckeinrichtung der eingangs genannten Art eine Vorrichtung bereitzustellen, die es ermöglicht von dem. Aufzeichnungsträger mitgeführte Partikeln abzufangen, so daß es zu keiner Beschädigung der Druckeinrichtung kommt. Die Vorrichtung soll einfach. und kostengünstig ausgestaltet sein und insbesondere die Papierführung nicht behindern.

Diese Aufgabe wird bei einer Schnelldruckeinrichtung der eingangs genannten Art gemäß den Merkmalen des ersten Patentanspruches gelöst.

Vorteilhafte Ausführungsformen der Erfindung sind in den abhängigen Ansprüchen gekennzeichnet.

Dadurch daß im Papierkanal der Druckeinrichtung in Papiertransportrichtung vor der eigentlichen Druckstation eine Partikelfalle zum Abfangen von mitgeführten Partikeln vorgesehen ist, können mit dem Aufzeichnungsträger mitgeführte Büroklammern oder andere Metallstücke die Druckstation nicht beschädigen.

Ein entsprechend der Größe der abzufangenden Partikeln dimensionierter Passierschlitz ermöglicht die störungsfreie Durchführung des Aufzeichnungsträgers, mitgeführte Partikeln jedoch verklemmen sich in dem Passierschlitz und führen zu einem Papierriß bzw. zu einer Unterbrechung des Papiertransportes.

Diese Unterbrechung des Papiertransportes wird mit Hilfe einer optoelektronischen Abtasteinrichtung erkannt und der Papiertransport im Drucker unterbrochen und z.B. eine Warneinrichtung auf dem Bedienungsdisplay aktiviert.

Bei einer vorteilhaften Ausführungsform der Erfindung enthält die Partikelfalle eine Stange in Form eines Partikelbalkens die gegenüber einem Umlenkprofil der Umdruckstation angeordnet ist. Von dem Aufzeichnungsträger mitgeführte Partikeln verklemmen sich zwischen Partikelbalken und Umlenkprofil.

Zum Entfernen der Partikeln kann das Umlenkprofil mit der Umdruckstation über eine Betätigungsschwinge von dem Partikelbalken abgeschwenkt und damit der Passierschlitz geöffnet werden.

Dies ermöglicht eine schnelle Wiederinbetriebnahme der Druckeinrichtung bei Druckunterbrechungen.

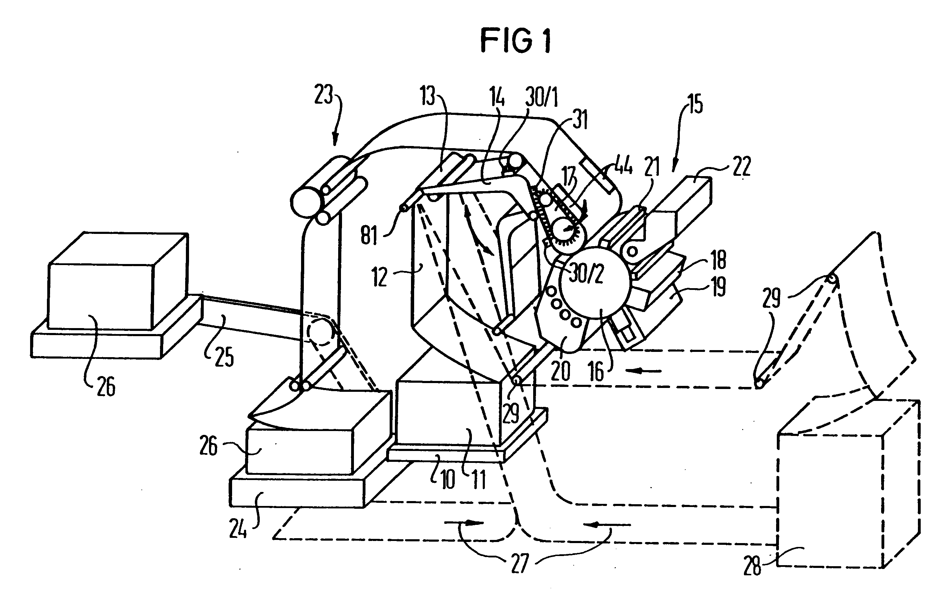

Eine Ausführungsform der Erfindung ist in den Zeichnungen dargestellt und wird im folgenden beispielsweise näher beschrieben. Es zeigen
FIG 1 eine schematische Darstellung einer elektrofotografischen Druckeinrichtung,
FIG 2 eine schematische Frontansicht einer Partikelfalle,
FIG 3 eine schematische Seitendarstellung einer Partikelfalle im Eingangsbereich des Papierführungskanales,
FIG 4 eine schematische Schnittdarstellung einer Umdruckstation einer elektrofotografischen Druckeinrichtung mit darin angeordneter Partikelfalle in verschiedenen Betriebszuständen und
FIG 5 eine schematische Schnittdarstellung der Umdruckstation gemäß FIG 4 im abgeschwenkten Zustand mit geöffneter Partikelfalle.

Eine nach dem Prinzip der Elektrofotografie arbeitende Druckeinrichtung weist einen Vorratstisch 10 zur Aufnahme eines Vorratsstapels 11 aus vorgefaltetem Endlospapier 12 auf. Das Endlospapier wird über eine Papierteilereinrichtung 13 und eine mit Papierleitelementen versehene, über einen Betätigungsgriff 81 mit Verriegelungseinrichtung abschwenkbare Betätigungsschwinge 14 dem eigentlichen elektrofotografischen Druckaggregat 15 zugeführt. Dieses Druckaggregat 15 weist eine, an eine Fotoleitertrommel 16 an- und abschwenkbare Umdruckstation 17 auf, sowie um die Fotoleitertrommel 16 angeordnete, für den elektrofotografischen Prozeß notwendige Einrichtungen.

Zur Erzeugung eines Tonerbildes auf dem Endlospapier wird in üblicher Weise die mit Hilfe einer Ladeeinrichtung 18 aufgeladene Fotoleitertrommel 16 zeichenabhängig über einen LED-Zeichengenerator 19 entladen, und das so erzeugte Ladungsbild in einer Entwicklerstation 20 mit einem Entwicklergemisch aus Toner- und Trägerteilchen eingefärbt. Das Tonerbild wird dann in der Umdruckstation 17 auf das Endlospapier 12 übertragen. Nach dem Umdruck wird die Fotoleitertrommel 16 über eine Entladestation 21 entladen und in einer Reinigungsstation 22 gereinigt und erneut über die Ladeeinrichtung 18 aufgeladen.

Anstelle des beschriebenen elektrofotografischen Prozesses ist es jedoch auch möglich zur Erzeugung des Tonerbildes auf dem Endlospapier 12 z.B. einen elektrostatischen Prozeß oder einen magnetischen Prozeß oder sogar einen Tintenkamm zu verwenden, der unmittelbar auf das Endlospapier Tinte aufbringt.

Die mit einem Tonerbild versehene Papierbahn 12 wird dann in einer Fixierstation 23 chemisch oder durch Wärme fixiert und auf einem Ablagetisch 24 abgelegt. Bei dem dargestellten Ausführungsbeispiel der Druckeinrichtung ist der Ablagetisch 24 über einen Schwenkhebel 25 zur Erleichterung der Entnahme des bedruckten Papierstapels 26 ausschwenkbar ausgestaltet.

Wird die Druckeinrichtung z.B. mit einer weiteren Druckeinrichtung gekoppelt um z.B. Vor- oder Rückseitendruck zu ermöglichen, kann die Papierbahn 12 auch über externe Papierzuführungskanäle 27 der Papierteilereinrichtung 13 unmittelbar zugeführt werden. Weiterhin ist es möglich als Vorratsstapel einen externen Endlosvorratsstapel 28 zu verwenden. Zur Zuführung der Papierbahn können dabei gesonderte Papierzuführungselemente mit Papierwalzen 29 notwendig sein.

Um das Eindringen von die Fotoleitertrommel 16 beschädigenden Partikeln wie Büroklammern oder anderen Metallteilen in das Druckaggregat 15 zu verhindern, kann am Eingangsbereich zur Umdruckstation 17 oder kurz vor dem Umdruckbereich eine Partikelfalle 30, 30/1, 30/2 angeordnet sein. Die Druckeinrichtung weist weiterhin eine über die Betätigungsschwinge 14 aktivierbare Papiereinlegevorrichtung mit zugeordneter Papierbremse 31 auf sowie als Papiertransportelemente Papiertraktoren 44, die vor und hinter der Umdruckstelle angeordnet sind.

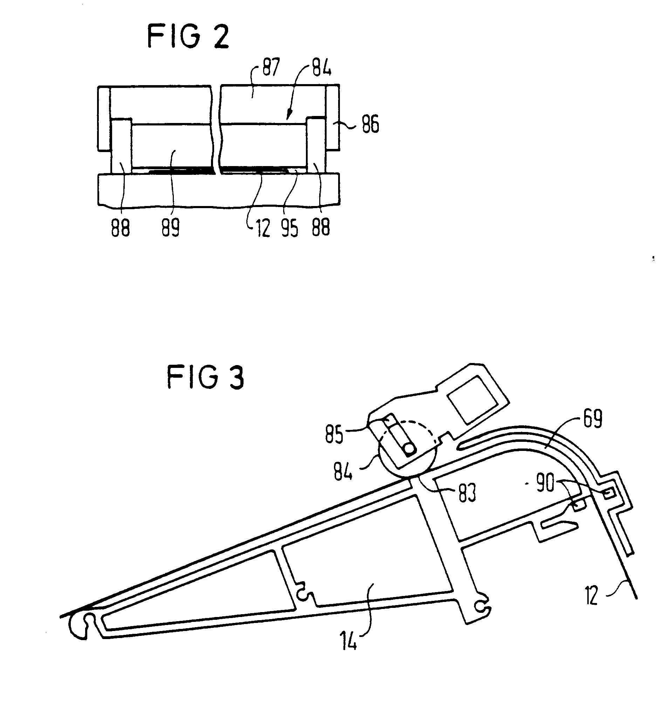

Die Partikelfalle gemäß dem Ausführungsbeispiel der FIG 2 und 3 enthält einen auf der Betätigungsschwinge 14 kurz vor dem Umlenkbereich 69 angeordneten Papiersattel 83 und eine runde den Papiersattel 83 entsprechend der Breite des Aufzeichnungsträgers 12 überspannende Metallstange 84 die in Schlitzen 85 einer seitlichen Halterung 86 geführt ist. Die Halterung ist über einer Querstange (Querprofil) 87, an dem Gehäuse der Druckeinrichtung befestigt. Die Metallstange 84 wiederum weist zwei seitliche runde Führungsbereiche 88 mit großem Durchmesser und einen sich quer über die Papierbahn erstreckenden Fangbereich 89 mit kleinerem Durchmesser auf. Dadurch ergibt sich zwischen dem Papiersattel 83 und der Metallstange 84 im Fangbereich 89 ein Passierschlitz für das Papier 12.

Ein in den Papierschlitz eingedrungenes Metallelement z.B. eine Büroklammer verkeilt sich im Passierschlitz und führt zu einem Riß der Papierbahn 12. Dieses Reißen der Papierbahn 12 wird über entsprechende Detektoren z.B. in Form einer im Umlenkbereich 69 angeordneten Lichtschranke 90 erkannt und der Papiertransport gestoppt. Zum Entfernen der Partikel kann die Metallstange 84 in den Schlitzen 85 der seitlichen Halterung 86 nach oben bewegt werden. Die Halterung 86 mit den Schlitzen 85 ist über die Querstange 87 in einem derartigen Winkel zur Papierbahn angeordnet, daß der Keileffekt beim Eindringen von Metallpartikeln unterstützt wird. Darunter ist zu verstehen, daß es sicher zu einem Verkeilen des abzufangenden Metallstückes mit nachfolgendem Papierriß kommt.

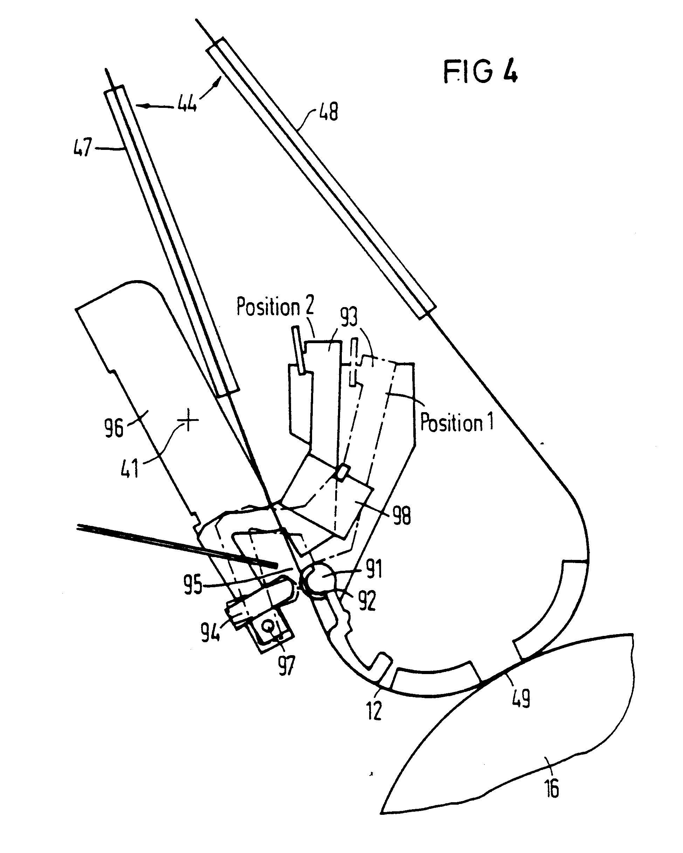

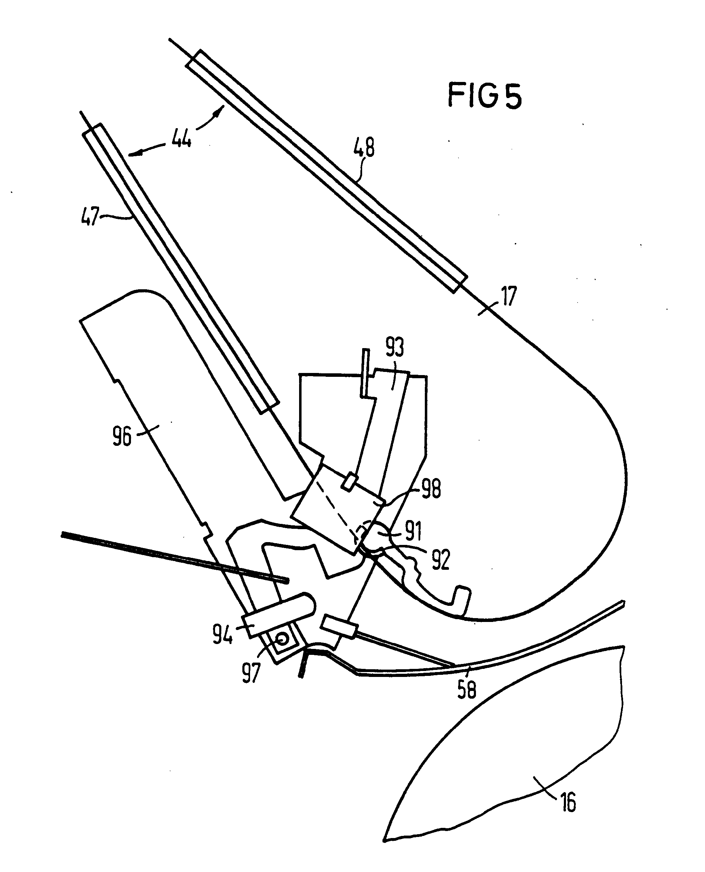

Bei einem bevorzugten Ausführungsbeispiel der Partikelfalle gemäß den FIG 4 und 5 ist die Partikelfalle auf der Umdruckstation 17 zwischen den eingangs- und ausgangsseitigen Papiertraktoren 44 in Papiertransportrichtung vor der Umdruckstelle 49 angeordnet. Damit kann sich bei einer Bewegung des Papieres 12 sowohl in Vorwärts- als auch in Rückwärtsrichtung das Papier 12 nicht in dem Passierschlitz 95 der Partikelfalle verklemmen, da das Papier 12 immer zwischen den Papiertraktoren 44 eingespannt ist.

Die Partikelfalle selbst enthält ein im Papierweg der Umdruckstation 17 angeordnetes Umlenkprofil 91, das sich über die Breite der Umdruckstation 17 erstreckt und auf dem das Endlospapier 12 mit seiner unbetonerten Rückseite gleitet. Auf dem Profil sind an den seitlichen Enden Abstandsbleche 92 befestigt. Damit ergibt sich eine Struktur für das Umlenkprofil 91, die der Metallstange 84 einschließlich der Führungsbereiche 88 des Ausführungsbespieles der FIG 2 entspricht.

An das Umlenkprofil über einen Hebel 93 anschwenkbar ist ein Partikelbalken 94 in Form einer Stange aus Metall, wobei in angeschwenktem Zustand der Partikelbalken 94 auf den Abstandsblechen 92 aufliegt und so zwischen Umlenkprofil 91 mit darauf geführtem Endlospapier 12 einen Passierschlitz 95 (Kalibrierspalt) zum Abfangen der Partikeln bildet.

Die Einlaufgeometrie in den Kalibrierspalt 95 ist trichterförmig ausgestaltet. Damit ist sichergestellt, daß es zu einem Verkeilen des abzufangenden Metallstückes mit nachfolgendem Papierriß kommt.

Der Hebel 93 ist auf einem Lagerbock 96 für das Schwenklager 41 der Betätigungsschwinge 14 gelagert und zwar um einen Drehpunkt 97 schwenkbar. Weiterhin weist er eine Rastvorrichtung 98 auf, die es ermöglicht den Hebel 93 in zwei Positionen zu verrasten. Diese Rastpositionen werden über einen Mikroschalter 99 abgetastet.

Mit Hilfe dieser Rastvorrichtung 98 läßt sich der Hebel 93 in zwei Positionen verrasten und zwar in einer ersten Position (Position 1), bei der der Partikelbalken 94 an das Umlenkprofil 91 angeschwenkt und damit die Partikelfalle geschlossen ist und in einer zweiten Position (Position 2), bei der der Partikelbalken 94 von dem Umlenkprofil 91 abgeschwenkt ist. Diese abgeschwenkte Position 2 ist notwendig, wenn mit der Druckeinrichtung auf dem Aufzeichnungsträger lösbar angeordnete Etikette bedruckt werden sollen, die sich ansonsten im Passierschlitz 95 verklemmen würden. Die verschiedenen Positionen des Hebels werden über den Mikroschalter 99 erkannt und der Gerätesteuerung mitgeteilt. Die Gerätesteuerung überwacht damit die Position des Hebels 93.

Bei beiden Hebelpositionen der FIG 4 befindet sich die Umdruckstation 17 in Betriebslage, d.h. in einer an die Fotoleitertrommel 16 angeschwenkten Position mit verrasteter Betätigungsschwinge 14.

Verkeilt sich in dieser Betriebslage im Kalibrierspalt 95 ein Metallstück, so reißt die Papierbahn und dieser Riß wird über die Lichtschranke 90 erkannt und der Papiertransport gestoppt. Zum Entfernen der Partikeln wird entsprechend der FIG 5 über die Betätigungsschwinge 14 die Umdruckstation 17 abgeschwenkt. Dadurch schwenken Papierleitelemente 58 in den Papiertransportkanal und schützen die Fotoleitertrommel 16. Aus dem Kalibrierspalt 95 beim Öffnen fallende Partikeln können damit die Fotoleitertrommel 16 nicht beschädigen.

Die Überwachung des Papiertransportes über die Lichtschranke 90 kann mit Hilfe der eigentlichen Gerätesteuerung der Druckeinrichtung erfolgen, die hier nicht dargestellt ist und die z.B. entsprechend der US-PS 4 593 407 ausgebildet sein kann. Wird der Papiertransport unterbrochen und gibt damit die Lichtschranke 90 ein entsprechendes Unterbrechungssignal an die Gerätesteuerung ab, so stoppt die Gerätesteuerung den weiteren Antrieb der Papiertransportelemente 44, die bei der vorliegenden Ausführungsform der Erfindung als Papiertraktoren ausgebildet sind. Die Unterbrechung wird auf einem entsprechenden Steuerungsdisplay dargestellt und die Bedienperson kann durch einfaches Abschwenken der Betätigungsschwinge 14 über einen Handgriff 81 das abgefangene Metallobjekt entfernen und über den durch das Abschwenken der Betätigungsschwinge entstandenen Einlegekanal das Endlospapier erneut einlegen.

## Patentansprüche

1. Schnelldruckeinrichtung, bei der einer Druckstation (15) über einen Papierkanal ein Aufzeichnungsträger (12) zugeführt wird und im Papierkanal in Papiertransportrichtung vor dem Druckbereich (49) eine Partikelfalle (30/1, 30/2) zum Abfangen von mit dem Aufzeichnungsträger (12) mitgeführten Partikeln angeordnet ist,
**dadurch gekennzeichnet,** daß die Partikelfalle (30/1, 30/2) als mechanische Partikelfalle ausgebildet ist mit einem entsprechend der Größe der abzufangenden Partikeln dimensionierten Passierschlitz (95) für den Aufzeichnungsträger (12) der einen Papiersattel (83, 91) und eine im Abstand zum Papiersattel (83, 91) angeordnete, den Aufzeichnungsträger (12) überspannende Fangstange (84, 94) aufweist.

2. Schnelldruckeinrichtung nach Anspruch 1, **dadurch gekennzeichnet,** daß der Papiersattel (83, 91) und/oder die Fangstange (84, 94) abschwenkbar ausgestaltet sind.

3. Schnelldruckeinrichtung nach Anspruch 1 oder 2,
**dadurch gekennzeichnet,** daß der Passierschlitz (95) eine im Querschnitt keilförmige Struktur aufweist, so daß mitgeführte Partikeln sich zwischen Fangstange (84, 94) und Papiersattel (83, 91) verklemmen.

4. Schnelldruckeinrichtung nach einem der Ansprüche 1 bis 3, **dadurch gekennzeichnet,** daß im Papierkanal eine Abtasteinrichtung (90) für den Aufzeichnungsträger (12) vorgesehen ist, die nach Feststellung einer Aufzeichnungsträgertransportstörung infolge einer abgefangenen Partikel in der Partikelfalle (30/1, 30/2) den weiteren Aufzeichnungsträgertransport unterbricht.

5. Schnelldruckeinrichtung nach Anspruch 4, **dadurch gekennzeichnet,** daß die Abtasteinrichtung (90) als optoelektronische Abtasteinrichtung ausgebildet ist.

6. Schnelldruckeinrichtung nach einem der Ansprüche 1 bis 5, **dadurch gekennzeichnet,** daß die Partikelfalle (30) auf der Umdruckstation (17) einer elektrofotografischen Druckeinrichtung zwischen eingangs- und ausgangsseitig angeordneten Papiertraktoren (44) vor dem Umdruckbereich (49) positioniert ist.

7. Schnelldruckeinrichtung nach Anspruch 1 oder 2,
**dadurch gekennzeichnet,** daß die Fangstange (84, 94) an einem verschwenkbaren, über Rastelemente (98) verrastbaren Hebel (93) angeordnet ist.

## Revendications

1. Dispositif d'impression rapide, dans lequel un support d'enregistrement (12) est envoyé à un poste d'impression (15) à travers un canal de circulation du papier et un piège à particules (30/1, 30/2), servant à collecter les particules entraînées simultanément par le support d'enregistrement (12), étant disposé dans le canal de circulation du papier, en amont de la zone d'impression (49) dans la direction de transport du papier,
caractérisé par le fait que le piège à particules (30/1, 30/2) est réalisé sous la forme d'un piège à particules mécanique, comportant une fente de passage (95) pour le support d'enregistrement (12), qui est dimensionnée en fonction de la taille des particules devant être collectées et qui comporte un support (83,91) pour le papier et une barre de collecte (84,94), disposée à distance du support (83,91) pour le papier et s'étend au-dessus du support d'enregistrement (12).

2. Dispositif d'impression rapide suivant la revendication 1, caractérisé par le fait que le support (83,91) pour le papier et/ou la barre de collecte (84,94) sont agencés de manière à pouvoir être écartés par pivotement.

3. Dispositif d'impression rapide suivant la revendication 1 ou 2, caractérisé par le fait que la fente de passage (95) possède une structure en forme de coin en coupe transversale de sorte que des particules entraînées conjointement se bloquent entre la barre de collecte (84,94) et le support (83,91) pour le papier.

4. Dispositif d'impression rapide suivant l'une des revendications 1 à 3, caractérisé par le fait que dans le canal de circulation du papier il est prévu un dispositif d'exploration (90) pour le support d'enregistrement (12), ce dispositif interrompant la poursuite du transport du support d'enregistrement lorsqu'il est établi qu'il existe une perturbation du transport du support d'enregistrement, due à une particule collectée, située dans le piège à particules (30/1, 30/2).

5. Dispositif d'impression rapide suivant la revendication 4, caractérisé par le fait que le dispositif d'exploration (90) est réalisé sous la forme d'un dispositif d'exploration opto-électronique.

6. Dispositif d'impression rapide suivant l'une des revendications 1 à 5, caractérisé par le fait que le piège à particules (30) est positionné sur le poste de transfert (17) d'un dispositif d'impression électrophotographique, entre les tracteurs à papier (44) disposés côté entrée et côté sortie, en avant de la zone de transfert (49).

7. Dispositif d'impression rapide suivant la revendication 1 ou 2, caractérisé par le fait que la barre de collecte (84,94) est disposée sur un levier pivotant (93), qui peut être encliqueté par l'intermédiaire d'éléments d'encliquetage (98).

## Claims

1. High-speed printing device in which a recording carrier (12) is fed to a printing station (15) via a paper channel and a particle trap (30/1, 30/2) for collecting particles entrained with the recording carrier (12) is arranged in the paper channel in the paper transport direction upstream of the printing area (49), characterised in that the particle trap (30/1, 30/2) is configured as a mechanical particle trap with a passing slot (95), which is dimensioned to correspond to the size of the particles to be collected, for the recording carrier (12) which has a paper saddle (83, 91) and a collecting rod (84, 94) which is arranged at a distance from the paper saddle (83, 91) and spans the recording carrier (12).

2. High-speed printing device according to Claim 1, characterised in that the paper saddle (83, 91) and/or the collecting rod (84, 94) are of swivel-away design.

3. High-speed printing device according to Claim 1 or 2, characterised in that the passing slot (95) has a structure which is wedge-shaped in cross-section so that entrained particles become jammed between collecting rod (84, 94) and paper saddle (83, 91).

4. High-speed printing device according to one of Claims 1 to 3, characterised in that in the paper channel a sensing device (90) is provided for the recording carrier (12), which sensing device interrupts the further recording carrier transport after determining a recording carrier transport fault resulting from a particle collected in the particle trap (30/1, 30/2).

5. High-speed printing device according to Claim 4, characterised in that the sensing device (90) is configured as an optoelectronic sensing device.

6. High-speed printing device according to one of Claims 1 to 5, characterised in that the particle trap (30) is positioned on the transfer station (17) of an electrophotographic printing device between paper tractors (44), arranged on the input side and output side upstream of the transfer area (49).

7. High-speed printing device according to Claim 1 or 2, characterised in that the collecting rod (84, 94) is arranged on a swivellable lever (93) which can be locked by means of locking elements (98).
